# EUROPEAN PATENT APPLICATION

(11) **EP 4 293 351 A1**
(43) Date of publication of application: **20.12.2023**
(21) Application number: 22187044.7
(22) Date of filing: 26.07.2022
(51) Int. Cl.: G01N 27/623, G01N 33/50, G01N 33/68

(54) **SENSITIVE DETECTION OF FOOD ALLERGENS BY IMMUNOENRICHMENT AND LC-MS/MS**

(30) Priority: 17.06.2022 DE 102022115203
(71) Applicant: ifp Privates Institut für Produktqualität GmbH, 12489 Berlin (DE)
(72) Inventor: ROEDER, Martin, 12489 Brelin (DE)
(74) Representative: Benedum, Ulrich Max

(57) **Abstract**

Sample preparation for the detection, and quantification, of food allergens by immunoaffinity enrichment of unique tryptic peptides of the allergen tested and their analysis by multiplex LC-MS/MS. The method according to the invention starts with comminuting, extracting, and dissolving the food sample in a chaotropic extraction buffer with pH > 8.0 at 40-95°C. The extraction buffer may contain more than 2 moles per liter of urea, thiourea, guanidinium chloride, guanidinium isothiocyanate, and an ammonium pentaborate salt that provides counterions for the extracted proteins. Extraction is followed by enzymatic degradation of the polymeric carbohydrates by an enzyme of category E.C. 3.2.1 likewise at 60 -95°C for at least 10 minutes, and optionally, reduction and alkylation of disulfide bonds. The extract is diluted with water and pH adjusted to 7.2 -7.9 to perform enzymatic sequence-specific proteolysis and/or trypsinization of the protein pool. The enzyme for the degradation of polymeric carbohydrates belongs to category E.C. 3.2.1.1, preferably α-amylase of *Bacillus licheniformis.* Immunoenrichment of one or more unique peptides of the target allergen provides higher sensitivity of subsequent multiplex LC-MS/MS.

## Description

### FIELD OF THE INVENTION

The present application relates to kits and methods for detecting and quantifying allergens in a sample using immunoaffinity enrichment and high-pressure liquid chromatography combined with mass-spectrometry for selected peptides **(G01N 27/623).**

### BACKGROUND OF THE INVENTION

Allergy and food allergies are common medical conditions. Up to 2% of adults and up to 8% of children, particularly those under three years of age, suffer from food allergies, and this prevalence is believed to be increasing. Food allergy is a potentially life-threatening immunological disorder caused by these hypersensitivities to specific food allergens. There is currently no remedy or cure other than avoidance of allergens. Multiple analytical methods are available to detect food allergens, including the enzyme-linked immunosorbent assay (ELISA), lateral flow devices, and liquid chromatography-tandem mass spectrometry. Commercial ELISAs are available for the most critical food allergens, which must be specified on all commercial food products per EU Regulation No. 1169/2011. The most critical foods and food ingredients to consider encompass: - (1) cereals containing gluten, wheat, rye, barley, Oats, Spelt, Kamut and products thereof, (2) crustaceans and products thereof; (3) eggs and products thereof; (4) fish and products thereof, (5) peanuts and products thereof; (6) soybeans and products thereof; (7) milk and products thereof, including lactose: (8) nuts in general, almonds, hazelnuts, walnuts, cashews, pecan nuts, Brazil nuts, pistachio nuts, macadamia or Queensland nuts, and products thereof; (9) celery and products thereof; (10) mustard and products thereof; (11) sesame seeds and products thereof; (12) sulfur dioxide and sulfites, if contained in specific higher concentrations; (13) lupin and products thereof; (14) mollusks and products thereof. The corresponding food labeling regulations in the U.S. are also quite detailed and comprehensive. To respond to the global non-communicable disease (NCD) crisis, the Codex Alimentarius Commission is developing an international guidance book for expanded front-of-pack nutrition labeling. Consequently, there will be a greater need for analytic tools to detect multiple allergens simultaneously.

Some specific edible foods and ingredients can be life-threatening when consumed by persons suffering from hypersensitivity to particular allergens present in these foods. Thus, they must strictly avoid these food allergens as there is no cure for food allergies. Therefore, there is further a need for food producers to detect the presence and/or verify the absence of these food allergens throughout the food production process.

Multiple analytical methods are available to detect food allergens, typically by immunological and molecular biology-based test formats. Still, tandem mass spectrometry is increasingly used for qualitative and quantitative detection of allergens. Chinese patent application No. 2018 10 745425.9 describes a method to extract the sesame seeds' allergens by an aqueous urea-thiourea solution containing a zwitterionic detergent, followed by trypsinization for 4 hours, de-salting, and analysis by liquid chromatography and tandem mass spectrometry. The sample preparation is laborious and cumbersome due to the desalting step. WO 2013/033713 (DH Technologies Development Pte Ltd., Singapore) claims a method of detecting an allergen in a sample, which comprises (i) adding a proteolytic enzyme to the sample to lyse at least a portion of the allergen present in the sample into a plurality of peptides; and (ii) using liquid chromatography-tandem mass spectrometry (LC-MS/MS) to determine whether at least one peptide is in the sample. This method needs a specific Multiple Reaction Monitoring (MRM) for each peptide to enable a reliable quantitation of the allergen. WO 2022/044072 (Shimadzu) combines this method with an information acquisition unit. An analysis program of the mass spectrometry result allows a plurality of peptides generated from one allergen to be detected using acquired information from various combinations of peptides.

Despite continuous improvements in detection methods, there is no universal superior technique, and each of the methods listed creates issues with compatibility across different assay formats. Immunological methods have the advantage of a higher sensitivity because the allergens can be extracted from the food matrix using detergents. However, the presence of detergents in the sample can strongly interfere with LC-MS/MS methods. On the other hand, not using detergents lowers the sensitivity of LC-MS/MS when the allergen is not extracted efficiently from the sample matrix. The present inventor has recently presented a principle for detecting ovalbumin allergen in a sample with higher sensitivity and by LC-MS/MS (Röder M et al., in Improved Sensitivity of Allergen Detection by Immunoaffinity LC-MS/MS using Ovalbumin as a Case Study, Foods 2021, 10, 2932). However, this principle cannot be applied to highly processed or baked food samples where a complete extraction of the allergenic proteins from the food matrix represents a problem.

Furthermore, quantifying the allergenic protein requires complete trypsinization of the extracted protein allergens, which requires either a pre-purified protein without inhibitory substances and/or substantial amounts of highly purified trypsin. This tedious and costly step is not universally consistent due to the large variety of food samples. Thus, the method needs to be improved to combine the advantages of antibody-based enrichment of allergen peptides and LC-MS/MS. State of the art, therefore, represents a problem.

### BRIEF DESCRIPTION OF THE INVENTION

The problems are solved and objects achieved by a method for the detection and quantification of food allergens in a sample of the food and/or beverage by immunoaffinity enrichment and LC-MS/MS of one or more unique peptides from the target allergen, comprising the steps of:-
comminuting, extracting, and dissolving the sample in an aqueous extraction buffer at an elevated temperature between 40 and 95 degrees Celsius to obtain an aqueous extract of said sample, said buffer having a pH greater than 8.0 and containing greater than 2 moles per liter of a chaotropic agent selected from urea, thiourea, guanidinium chloride, guanidinium isothiocyanate, and further comprising a salt which provides inert counteranions for the dissolved peptides, amino acids, and carbohydrates, said counteranions being incapable of reacting with or precipitating calcium ions;
degradation of the polymeric carbohydrates present in the aqueous extract by adding an adequate amount of an enzyme of category E.C. 3.2.1 at an elevated temperature between 40 and 95 degrees Celsius for at least 10 minutes;
reduction of intra- und intermolecular peptidic disulfide bonds, followed by the addition of water to dilute the chaotropic agent in the extract and an adjustment of the pH of the extract in the range from 7.2 to 7.9 for enzymatic proteolysis of the peptides;
addition of an adequate amount of a protease and proteolysis of the proteinaceous components in the extract; and
Immunological enrichment of one or more unique analytical peptides of the target allergen for higher sensitivity of the subsequent multiplex LC-MS/MS.

In some preferred embodiments, the enzyme for the degradation of polymeric carbohydrates is of category E.C. 3.2.1.1, preferably α-amylase of *Bacillus licheniformis.*

In some more preferred embodiments, the extraction buffer may contain added calcium ions for increased stability of the α-amylase. Alternatively, or additionally, the extraction buffer may contain borate counter ions provided from a pentaborate salt, preferably from ammonium pentaborate.

In a most preferred embodiment, the extraction buffer is a borate buffer having a pH range from 8 to 10. The extraction buffer can be free of anionic or zwitterionic detergents.

In some preferred embodiments of the method, the chaotropic agent in the extraction buffer is urea.

In some embodiments, the protocol contains a step before proteolysis or trypsinization of the extracted protein pool, wherein the extraction buffer is diluted with an aqueous solution containing a buffer salt selected from ammonium bicarbonate or ammonium acetate for pH adjustment

In some preferred embodiments, the extracted protein pool and the food allergens are reduced and alkylated before sequence-specific proteolysis or trypsinization.

In some most preferred embodiments, trypsin is used for specific proteolysis.

In other embodiments, an alternative protease is used for proteolysis selected from aspartic proteases, napsin-A, pepsin, presenilin, chymosin, cathepsin D/E, glutamyl endopeptidase (Glu-C), elastase, chymotrypsin, facultatively in a sequential combination with trypsin.

In accordance with the invention, the immunological enrichment of the target allergen's unique or specific proteolytic peptides or proteins comprises an immunoaffinity chromatography, preferably using one or more monoclonal antibodies specifically binding linear epitopes or peptides. The person skilled in the art will appreciate that this is a step in the method of the invention wherein the target peptides could eventually be eluted from the affinity column using an organic aqueous buffer or buffer containing a sublimable elution buffer, in which case there is the additional advantage that no desalting of the sample is needed prior LC-MS/MS.

Another aspect of the invention relates to a kit of parts for carrying out a food allergen extraction, comprising:-
a detergent-free extraction buffer having a pH of greater than 8.0, containing more than 2.0 Moles per liter of a chaotropic agent selected from urea, thiourea, guanidinium chloride, guanidinium isocyanate, and a salt providing counterions for dissolved proteins, amino acids, and carbohydrates which do not react with or precipitate calcium ions; and
a solution of α-amylase of *Bacillus licheniformis.*

In some embodiments, the kit of parts may further comprise an ammonium carbonate buffer for pH adjustment of the protein extract and an active trypsin solution for sequence-specific cleavage of dissolved proteins.

In some embodiments, the kit of parts may further comprise an immunoaffinity column for a unique tryptic peptide of the selected target allergen. The immunoaffinity column may contain a bound monoclonal antibody binding a unique peptide of the food allergen being tested.

### A DETAILED DESCRIPTION OF THE INVENTION

Immunoassays have been used for decades to detect protein allergens. Standard methods include sandwich- or competitive ELISAs, lateral flow immunochromatography, or antibody-based SPR spectroscopy. Quantitative immunoassays generally require internal calibration, and suppliers of these assays typically mandate the use of proprietary calibrators and/or standard matrices for measurements. This is incompatible with a regulatory framework that must be universal and not dependent on a particular test provider. In addition, immunological allergen determinations are unreliable because they rely on binding specific protein epitopes. However, these may be entirely or partially lost during food processing or sample preparation. In addition, epitopes can go lost while allergen-relevant epitopes are retained during food processing or sample preparation. The immunological methods are further dependent on the properties of the antibodies used, their affinity and specificity, and a complete release of the target allergen from the sample matrix. Disadvantageously, monoclonal antibodies preferably have a three-dimensional nonlinear epitope as a specific determinant because they can bind that with higher affinity than simple linear epitopes, but three-dimensional epitopes are temperature sensitive, and they require a suitable buffer environment. PCR-based assays could be used to detect the genes encoding the specific allergens. This approach allows quantitative determination only in exceptional cases or after complex standardization. It requires the presence of sufficient quantities of the encoded DNA, which is not always the case for allergenic foods such as milk (casein) or egg.

LC-MS/MS peptide mapping of allergens would be an attractive alternative to the methods above. Still, the sensitivity of this technique is too low to detect traces of food allergens in complex or processed foods. According to the present disclosure, this problem is solved by providing a universal workflow for completely releasing food allergens from all kinds and types of foods. This is not a simple step due to the variability and different compositions of the sample matrices. More specifically, food allergens must first be quantitatively and reliably released from highly complex food matrices. Secondly, they must be recovered in a dissolved form that allows quantitative digestion - not just partial digestion - so that, thirdly, specific linear peptides of the target allergen can be immunologically enriched in the sample, which can then be analyzed with high sensitivity by multiplex LC-MS/MS. Additionally, the workflow must further be translatable into automated processing.

The workflow starts with extracting proteins from the sample, which must be uniform, representative, and homogenous. The initial sample preparation from solid and packaged foods, prepared and ready-to-eat foods and dishes start with intensive comminution and homogenization and mixing with a chaotropic buffer. The food allergens must also be released from a complex food matrix and brought into a dissolved form. The complex food matrix may be a highly processed, thermally treated food containing various diverse ingredients, such as nutritional and candy bars, toppings, pickles, canned soups, prepared meals, etc. These often contain large amounts of a wide variety of different polymeric carbohydrates for texture and nutritiousness. The comminuted and homogenized sample is therefore subjected to enzymatic degradation of these polymeric carbohydrates using an enzyme of category E.C. 3.2.1 such as α-amylase in a chaotropic buffer at an elevated temperature in the range of 60 to 98 degrees Celsius, simultaneously or in a sequential combination of process steps. This pretreatment and treatment all complete extraction of proteins from the food matrix, even from highly complex food matrices, and the resulting extract of pooled proteins and dissolved food allergens can then be readily adjusted to pH and diluted to become subjected to proteolytic digestion in a one-pot reaction, without intermediate purification and protein loss. The extracted sample then contains unique linear peptides of the target allergens. The combined lysis of polymeric carbohydrates also allows lower protease amounts for complete digestion of the food allergens, reducing enzyme costs considerably and enabling routine quantitative determinations of allergens in foods, where sample sizes and protein amounts are comparatively large compared to other analytical processes using protein mapping by LC-MS/MS.

The concomitant degradation of linear and branched long-chain polymeric carbohydrates is critical because many foods contain starch, amylopectin, and/or modified starches in varying and often abundant amounts. We have found that the polymeric carbohydrate structures impede the release and solubilization of food allergens even from highly comminuted and homogenized food samples. They also inhibit proteolytic digestion and trypsinization by the entanglement of enzymes. In particular, "modified starch" represents a problem. According to the FAO Food and Nutrition Program (FNP), this term describes starch in which one or more of its original physicochemical properties have been altered by hydrolysis, esterification, etherification, oxidation, and/or cross-linking. When starch is heated with acid or alkali, fragmentation occurs. The oxidation and bleaching of starch introduce carboxyl groups which may react with amines and amino acids of the food allergens. Treatment with bifunctional substitution reagents such as orthophosphoric acid leads to a substitution in the 2-, 3- or 6-positions of the anhydroglucose unit (AGU) and a cross-linking. Enzymatic treatments and/or genetic modifications are also used to obtain starch-cyclomaltodextrin complex granules that provide flavors and aromas. Typical examples are amylomaltases or α-1,4-α-1,4-glucosyltransferases from *Thermus thermophiles* and cyclomaltodextrinase (CDase 1-5) from the *alkalophilic Bacillus sp.* While α-1,4-α-1,4-glucosyltransferases cleave existing α-1,4-bonds and produce new bonds, CDase 1-5 is used to obtain low-amylose starches without altering the amylopectin distribution. Transglucosidase and malt-forming α- and β-amylases are used to produce resistant starches.

Furthermore, glycine and proline can react with starch at relatively low temperatures. The alkaline amino acids arginine, histidine, and lysine can react as bifunctional reagents with starch at temperatures from 150 to 200°C. These so-called Maillard reaction products may also include sulfur atoms in the case of reactions with cysteine. Therefore, thermally treated foods contain Maillard reaction products that complicate the enzymatic carbohydrate breakdown. In addition, caramelization products, dehydration products, hydration products, disproportionation products, and some aromatic species are formed when the food or its ingredients have been baked, roasted, barbecued, or fried. Consequently, industrially produced foods and shelf-stable packaged foods may contain a wide variety of modified long-chain polymeric carbohydrates and starches with different gelling properties, which have reacted with or encapsulated or interacted with food allergens.

Therefore, the workflow includes a chaotropic treatment and dissolution of the complex food sample at elevated temperatures in the range of 60 to 98°C, most preferred 80 and 90°C, in combination and simultaneously with enzymatic degradation of starch, amylose, amylopectin, modified starch and reaction products of homopolysaccharides and polymeric carbohydrates using a glycosylase of category E.C. 3.2.1 that hydrolyzes O- and S-glycosyl compounds. Enzymes of category E.C. 3.2.1 hydrolyze naturally but incompletely starch, glycogen, and related polysaccharides and oligosaccharides. Most enzymes of category E.C. 3.2.1 can only act randomly on starch as an endohydrolase when starch molecules are elongated and granules uncoiled. The present inventors have found that a complete breakdown of polymeric carbohydrates - including amino acid-modified and peptide-modified polymeric carbohydrates - can be achieved by an enzymatic digest at elevated temperature when an enzyme is used that is stable and active above 60°C even in an aqueous chaotropic buffer environment.

In some embodiments, the enzyme of category E.C. 3.2.1 is amylase. Amylases can be obtained from plants, animals, and microorganisms. However, for costs, reliability, and availability, α-amylases from fungal and bacterial sources are preferred. Many microbial α-amylases are used in industrial sectors to produce textiles, paper, detergents, and foods. They, therefore, have known properties in terms of thermal stability, pH profile, pH stability, and calcium independence. Preferred is an α-amylase (α-1,4-glucan-4-glucanohydrolase) that catalyzes the cleavage of α-D-(1-4) glycosidic bonds. Neither terminal glucose residues nor α-1,6-linkages can be cleaved by α-amylase. The end products of α-amylase action are, therefore, oligosaccharides with varying lengths having an α-configuration and α-limit dextrins, the branched core of the polysaccharide fragments of amylopectin or glycogen. The main end-products, however, are a mixture of maltose, maltotriose, and branched oligosaccharides of 6-8 glucose units that contain both α-1,4 and α-1,6 linkages. Other amylolytic enzymes may be used in the starch breakdown, but α-amylase is the most important. The most prominent microbial sources for enzymes of category EC 3.2.1 are Aspergillus, Penicillium, Bacillus, and notably B. licheniformis (ATCC 27811; B4-423; etc.), B. subtilis, Brevibacillus, E.coli, Lactobacillus, Saccharomyces, S. cerevisiae, Thermococcus, Thermomyces, etc. α-Amylase may also be isolated from animal and mammalian sources (saliva, pancreas, etc.). However, for cost reasons, enzymes from bacterial and fungal sources and recombinant α-amylase from sources generated by genetic engineering are preferred. The *Bacillus licheniformis* α-amylase [E.C. 3.2.1.1] is most preferred and an endo-hydrolase that randomly cleaves α-(1,4)-glycosidic bonds in polysaccharides containing three or more α-(1,4)-linked glucose units (Dona AC et al., Digestion of starch: In vivo and in vitro kinetic models used to characterize oligosaccharide or glucose release. Carbohydrate Polymers 210, 80(3):599-617). The term 'alpha' refers to the α-anomeric configuration of the liberated sugar or oligosaccharide. Alpha-amylases generally require calcium ions for their activity and structural integrity at elevated temperatures. For this reason, calcium ions may be added to the extraction buffer to facilitate the stability of the amylase enzyme at elevated temperatures. However, the endogenous calcium contained in the extracted food sample should generally be sufficient unless the calcium ions are precipitated or removed from the lysed extract. The combination of elevated temperatures of 60 to 98 degrees Celsius, mildly chaotropic buffer conditions, and the presence of calcium allows α-amylases to degrade natural starch of all kinds and sources and degrade modified starch and reacted polymeric oligosaccharides present in foods.

In some preferred embodiments of the invention, the extraction buffer is phosphate-free to avoid the formation of calcium phosphate precipitates at elevated temperatures. Preferably, the extraction buffer is free of any molecules that form stable complexes with calcium ions or can chelate the calcium ions. Since multivalent counterions are needed for good solubilization and dissolution of the extracted proteins and allergens in the chaotropic extraction buffer, a borate buffer may be used to keep the calcium ions of the sample actively in solution. The extraction buffer preferably contains ammonium borate to keep the calcium ions in solution - and to solubilize the proteins and food allergens - so that the enzymatic degradation of starch and modified polyhomosaccharides by the enzyme amylase is not disturbed or inhibited by the withdrawal of the calcium ions and the resulting lack of amylase stability and activity.

It is agreed that amylase is one of the most important enzymes used in industry, which is used to hydrolyze starch into smaller polymers of glucose units. A-amylases are also used in the baking industry. They are added to the bread dough to break down the starch in flour into smaller oligosaccharides and dextrins so that they are more readily fermented by yeast. However, the prior art does not disclose the use of α-amylase at elevated temperatures in a denaturing buffer system to degrade Maillard reaction products, natural and modified starch, and other polymeric carbohydrates contained in a complex food sample, thereby improving the reliability and sensitivity of food allergen detection. Release, solubilization, and trypsinization/proteolysis of food allergens must be quantitative to allow quantitative analysis and higher sensitivity through immunoaffinity enrichment for multiplex LC-MS/MS analysis.

In accordance with the present invention and workflow, the proteolytic digestion or trypsinization of the food allergens overcomes the problem that harsh extraction procedures can destroy native tertiary allergen structures, which prevents their detection and determination in conventional immunological methods [Walczyk NE et al. in Peanut protein extraction conditions strongly influence the yield of allergens Ara h 1 and 2 and sensitivity of immunoassays, Food Chem 2017, 221, 335-344; Ito K et al. in Food allergen analysis for processed food using a novel extraction method to eliminate harmful reagents for both ELISA and lateral-flow tests, Anal Bioanal Chem 2016, 408, 5973-5984; Lidzba N et al. in Development of monoclonal antibodies against pea globulins for multiplex assays targeting legume proteins, J Agric Food Chem 2021, 69, 2864-2874; Schubert-Ullrich P et al. in Commercialized rapid immunoanalytical tests for determination of allergenic food proteins: An overview, Anal Bioanal Chem 2009, 395, 69-81). The degradation of any starch and polymeric carbohydrates in the food sample greatly facilitates the trypsinization of the protein pool and/or further proteolytic digestions. It allows for reducing the ratio of protease (trypsin) to protein to 1:100 (w/w) or less and using regularly purified proteases. This is a significant saving, as trace allergen detection must start from a homogenous or comminuted sample of a few grams, the size of which cannot be reduced without losing sample homogeneity and sensitivity. Furthermore, since the extractable protein content is unknown, a considerable excess of MS-grade protease/trypsin must be added to the sample to ensure complete sequence-specific digestion. The sample size is not a cost factor for disulfide bond reduction and alkylation, but the costs of MS-grade protease/trypsin can be prohibitive for routine testing. However, proteolytic digestion or trypsinization must be complete to allow immunological enrichment and assignment of peptides by LC-MS/MS.

Trypsin is the protease of choice to cleave allergens into smaller fragments or peptides. Other proteases used alone or in sequential digestion steps are Asp-N, Glu-C, Lys-C, chymotrypsin, elastase, and proteinase K. However. In contrast, a digestion step with a complementary protease could potentially benefit two-step proteolysis; trypsinization usually generates sufficient peptides from the targeted food allergens for detection and quantitative evaluation. Therefore, trypsinization and tryptic peptides are used herein as generic terms or synonyms for all kinds of single- and double-step proteolysis of allergens and peptides obtained by proteolysis.

The generated samples are free from detergents after trypsinization/proteolysis and ready for immunological enrichment of selected detection peptides using immunoaffinity methods. In essence, the claimed protocol and workflow greatly simplify sample preparation for all food samples. They contain no steps that could lead to peptide loss or induce bias towards a particular class of peptides.

The sequence-specific detection peptides can be enriched in the sample to any degree using immunoaffinity methods. Thus, the present invention overcomes the reliability problem of immunological methods by analyzing sequence-specific peptides from food allergens and the sensitivity problem of current LC-MS/MS methods by a simple immunological enrichment of peptides that present a linear epitope. The use of monoclonal antibodies recognizing specific linear peptides is preferable for unlimited supply, availability, and reproducibility.

The present application provides a routine sample preparation protocol for most, if not all, food samples that combine immunoaffinity purification and mass spectrometry with taking advantage of the best aspects of antibody- and MS-based detection methods. The present invention thereby overcomes the drawbacks of both ELISA and conventional MS methods, as previously reported (Becker JO et al. in Replacing immunoassays with tryptic digestion-peptide immunoaffinity enrichment and LC-MS/MS, Bioanalysis 2012, 4, 281-290). ELISA and LC-MS/MS analyses also begin with extracting and solubilizing food allergens from the food matrix. However, the analytical advantage and the "sensitivity" of mass spectrometry decline with an increased protein content of the food matrix, in contrast to immunoaffinity methods which have the benefit of higher sensitivity. On the other hand, the immunoaffinity methods often have problems detecting food allergens with specificity and sensitivity in processed foods or after a harsh extraction of the sample matrix. The present invention overcomes these sensitivity and selectivity problems by focusing on the enrichment of linear tryptic peptides after trypsinization of the extracted food allergens. However, immunoenrichment of tryptic peptide requires, first, complete and universal extraction and solubilization of proteinaceous food allergens even from complex, polycarbohydrate-rich, and/or processed food samples and, second, complete proteolytic/tryptic digestion of the solubilized allergens in a buffer, which, third, also allows selective enrichment of tryptic peptides using immunoaffinity methods and highly specific detection of proteolytic/tryptic peptides using multiplex LC-MS/MS methods. The person skilled in the art will appreciate that this is a purification and enrichment step that may allow elution of the target peptides from the affinity column using an organic aqueous buffer or a buffer containing sublimable buffer salts, in which cases there is the additional advantage that no desalting of the sample is needed prior LC-MS/MS and which also allows a more straightforward analysis of the tryptic peptides by LC-MS/MS.

The protocol according to the invention is based on the fact, to be established first, that the proteolytic/tryptic peptides of the allergen and determined by LC-MS/MS are characteristic and unique to the target allergen so that comparable stable isotope-labeled (SIL) peptides can be used as an internal standard for their absolute quantitative determination in the food sample. With LC-MS/MS analysis, there is no risk of error or wrong determination due to antibody cross-reactivity. However, LC-MS/MS analysis requires complete extraction and tryptic digestion of the allergen and enrichment in the sample under investigation to be an alternative to purely immune-based detection and quantification. This is not easy, considering that the different and complex sample matrices also vary. According to the invention, the universal protocol is suitable for all processed and cooked food and feed types.

With the invention, the method's sensitivity method is no longer limited because the loading volume of the immunoaffinity columns for the specific tryptic peptide can be adjusted to the desired enrichment. Thus, the detection limits can be shifted to very low concentrations. Thus, the protocol of the invention also allows quantitative detection of allergens in substantial sample sizes, especially when food labels indicate to be "free of ... (a specified allergen)." While there is more than one peptide enrichment strategy, the immunoenrichment of specific peptides after tryptic digestion is particularly suitable because it results in a nearly pure peptide mixture that LC-MS/MS can readily analyze. The absence of more than 90% of competing peptide fragments and interfering metabolites or by-products results in a high S/N ratio. This improves sensitivity while avoiding MS instrument contamination and associated cleaning and maintenance costs. Furthermore, the described sample preparation and degradation of polymeric carbohydrates offers additional advantages such as shorter digestion times, a lesser amount of digestive enzyme, and a more straightforward immunoaffinity procedure.

On the other hand, starches in native and/or modified forms and in varying amounts are added to bakery products, confectionery, gravies, soups and sauces, mayonnaises, salad dressing, and ice cream, soft drinks, sausages, jams, dips, and so on. We have found that they strongly interfere with allergen extraction and subsequent trypsinization of the extracted proteins and allergens. Starch in its native form has only limited functionality. Therefore, a wide range of modified starches with different and improved physicochemical properties have been developed and are then added to foods for many reasons. The most common modification of starch is chemical, using the chemical reactivity of the hydroxy groups of the glucose monomers. Polysaccharides such as starch can be chemically modified by hydrolysis, esterification (e.g., starch succinate, starch citrate), etherification (e.g., hydroxy propylated starch - HPS, hydroxy ethylated starch - HES, cationic starch - a starch-containing electron-withdrawing groups such as ammonium, amino, imino, sulfonium, phosphonium), phosphorylation, oxidation, and cross-linking.

Starch must be gelatinized to be digestible by humans. On the other hand, starch gelatinization takes time because the starch globules must first swell and become extended in water to form elongated homopolysaccharide molecules, as happens, for example, when cooking corn flour porridge. The chemical modifications shorten the swelling time and result in a kind of pregelatinized starch. The gelatinization time may also be reduced by physical methods such as extrusion, spray drying, and drum drying. These methods are generally used to obtain flours and/or pre-gelatinized starches with long-term stability that allow faster preparations. This also affects the food qualities and properties, such as bread volume and crumb; pasta elasticity and softness, lusciousness and digestibility, tolerance in the properties of beating and cake mixtures, ice creams, doughnuts, growth of sugar crystals in food products; texture, volume, shelf-live and stability during thawing of cakes and loaves of bread. Liquefaction, partial hydrolysis, and dextrinization may occur during pre-gelatinization, depending on the processing conditions. Consequently, the amount of native or modified starch in a sample matrix significantly affects the extraction of food allergens and their detection and quantification in the sample matrix. Therefore, the starch and/or its chemically modified counterparts must first be degraded to allow complete extraction and liquification of the sample before any trypsinization or proteolysis of the food allergens can occur and/or any immunoaffinity reaction and enrichment of the unique target peptides from the allergen. The chemically modified starch can also impact the presentation of epitopes and food allergens even when those have not been chemically attached to the modified starch, in addition to the risk of a chemical reaction with the food allergen.

Finally, disulfide bridges between cysteines are fundamental elements of molecular architecture that can prevent the complete trypsinization/proteolysis of food allergens. These disulfide bonds can form intramolecularly between two adjacent cysteines or intermolecularly between cysteines of different peptide strands, sometimes resulting in nonspecific increased protein aggregation. In the present protocol, disulfide bonds are reduced and alkylated before trypsinization/proteolysis to prevent rebonding. The chemical reduction and alkylation are preferably done immediately before trypsinization or proteolysis, in practice, after degradation of the native or modified polymeric carbohydrates but can also occur at any time during extraction and starch degradation.

The sample preparation protocol and rationale for the various steps have been discussed in detail above. Further advantages and embodiments of the invention will become apparent from the examples below. It is understood that the above features and those explained in the following examples may be used in the combination indicated in each case, but also in other combinations or alone, without departing from the present invention.

### EXAMPLES

### Example 1 - Allergen extraction from the sample matrix

3 grams of food sample (cookies, cake, dough, sausage, etc.) were weighed into a 50-mL Falcon tube. The proteins were extracted from this sample and resolved by finely grinding the sample matrix and homogenizing it at room temperature in 30 ml of a chaotropic extraction buffer containing 2.5 M urea, 10 mM (NH₄)B₅O₈, 16 mM Tris-HCl, pH 8.5. To increase the overall sensitivity of the method for subsequent LC-MS/MS, the structural carbohydrate matrix was concomitantly broken down, thereby achieving complete extraction and solubilization of the protein allergens. This was done by (i) heating the homogenized mixture to 90°C for 10 minutes and (ii) enzymatic digestion of the polymeric carbohydrates and polyhomosaccharides in the food sample. For this purpose, 0,5 mL of α-amylase type XII-A (1,4-α-D-Glucan-glucanohydrolase) from *Bacillus licheniformis* (1 mg ≥500 units/mg protein) was added, and the polymeric carbohydrates were enzymatically digested with gentle agitation of the tube. The tube with the peptide sample was then cooled to room temperature and centrifuged at 4°C for 30 minutes at 4700 × g to separate insoluble or fibrous components from the supernatant containing the solubilized peptide allergens extracted from the complex food sample. Using the chaotropic buffer ensured that the peptide allergens were solubilized in the buffer and the starch enzymatically degraded by hydrolysis.

The *Bacillus licheniformis* α-amylase [E.C. 3.2.1.1], an endo-hydrolase that randomly cleaves α-(1,4)-glycosidic bonds in polysaccharides containing three or more α-(1,4)-linked glucose units. Alpha-amylases commonly require calcium ions for activity and structural integrity, so the presence of phosphate anions was avoided in the aqueous alkaline extract. The presence of natural or added calcium ions in the section contributes significantly to the stability of the amylase, in particular at elevated temperatures (Nazmi A et al., Calorimetric studies on renaturation by CaCl2 addition of metal-free α-amylase from Bacillus Licheniformis (BLA) in J Thermal Analysis and Calorimetry 2008, 91(1): 141-149). The precipitation of calcium phosphate in the alkaline extract having a pH greater than 8,0 can be approximately represented as the precipitation of hydroxyapatite:-

5Ca²⁺ + 3 PO₄³⁻ + OH⁻ = Ca₅(PO₄)₃OH ↓ (solid)

However, the precipitation of calcium phosphates is very complex, namely in the form of hydroxyapatite (HAP), dicalcium phosphate dihydrate (DCPD), dicalcium phosphate anhydrous (DCPA), and monocalcium phosphate monohydrate (MCPM. While the precipitation kinetics have been the subject of numerous studies and remains under dispute, it is clear that the calcium phosphate formed depends significantly on temperature conditions and compositions of buffer and sample. When working at an alkaline pH 8 or higher and temperatures above 60 degrees Celsius, precipitations can likely remove 90 percent or more of the soluble calcium ions when phosphate is present in the extract. The presence of phosphate ions should be avoided when using amylase enzymes of category E.C. 3.2.1, such as α-amylase from *Bacillus licheniformis.*

This extraction protocol works well for all kinds of foods, ready-made and canned meals, sausages, ground meat products, puddings (black, white, sweets, breakfast cereals, prepared dishes, food bars), energy bars, and candy bars, protein bars, snacks, etc. For comparison purposes, the inventors extracted peptide allergens from some backed sample matrices containing varying amounts of starch. The results have been summarized in **Table I.**

**TABLE 1**

| *Peptide allergen extraction from food samples with a variable starch content* | | | | |
|---|---|---|---|---|
| **Percentage (w/w) maize starch** | | **Amount of natural maize starch** | | **Sample solution after extraction with 30 ml chaotropic buffer and digestion of homopolysaccharides and amylopectin Extraction: 10min at 90°C, followed by 30 min at 4°C and centrifugation at 5000rpm** |
| | | | | **No degradation of homopolysaccharides** |
| | 10 | | 0,3 g | Clear, easily pipettable supernatant (about 22 mL) |
| | 20 | | 0.6 | Clear, easily pipettable supernatant (about 14 mL) |
| | 30 | | 0.9 | Clear, easily pipettable supernatant (about 6 mL) |
| | 40 | | 1.2 | Barely clear supernatant (1 to 2 ml), milky and viscous, pipettable |
| | 50 | | 1.5 | No clear supernatant, milky and viscous, hardly pipettable |
| | 60 | | 1.8 | A highly viscous solid gel that cannot be analyzed |
| | 70 | | 2.1 | Not analyzable for peptide allergens |
| | 80 | | 2.4 | Not analyzable for peptide allergens |
| | 90 | | 2.7 | Not analyzable for peptide allergens |
| | 100 | | 3.0 | Not analyzable for peptide allergens |
| | | | | |

| | | | | **With enzymatic degradation of starch by amylase** |
|---|---|---|---|---|
| | 100 | | 3,0 | Small pellet, turbid supernatant, pipettable, and analyzable for peptide |

Table I confirms that samples containing more than 40 percent by weight starch or amylopectin cannot be analyzed for peptide allergens. Even a minor starch content in the food sample can interfere with a quantitative peptide extraction from the food matrix and interfere with the peptide allergen detection and quantitation in foods.

Thus, the general conclusion remains that packed products such as cookies, cakes, and dough with added starch that tend to gelatinize, mainly when higher temperatures are used, are analyzed for food allergens. These samples are troublesome in further handling, as there is often not enough liquid supernatant available for the subsequent steps.

Although there are numerous methods for degrading polymeric carbohydrates, enzymatic degradation with amylase is preferred. However, it should be noted that starch and raw starch granules from different plant sources (potato, sweet potato, wheat, rice, and corn/maize) are different. Amylases break down these starch granules differently, with α-amylase being more efficient than beta-amylase. Alpha-amylases typically exhibit centrifugal and centripetal hydrolysis on corn, rice, and wheat starch granules but only centrifugal hydrolysis on potato starch granules. On the other hand, beta-amylase moves very slowly on the granules, and kinetic analyses show a maltose release. The rice starch granules are a suitable substrate for enzymatic hydrolysis by alpha- and beta-amylases. Furthermore, some beta-amylase can hydrolyze corn granules efficiently at 45°C, whereas soybean beta-amylase is 60% less active than bacterial beta-amylases at the same temperature. Thus, there is a wide variety between amylases, and the starch structure is also crucial for the degradation process. The present example overcomes these problems by an enzymatic degradation using an α-amylase in a slightly chaotropic environment, say at an increased temperature of 90°C in the presence of some urea, so that the starch granules are decoiled or unfolded regardless of the botanical origin of the starch - and fully accessible to any amylase.

### Example 2 - Trypsinization of protein allergens

For tryptic digestion, 5 ml of supernatant with extracted protein allergens was transferred to a 15 ml tube and mixed with 5 ml buffer containing 200 mM ammonium bicarbonate to adjust the pH to about 7.8 and lower the chaotropic activity of the urea present in the supernatant. Proteins were further treated and unfolded by adding 0.5 mL of 200 mmol DL-dithiothreitol to reduce disulfide bridges. This mixture was incubated for 45 minutes at room temperature and then alkylated by adding 0.5 mL of 400 mmol iodoacetamide, followed by 45 minutes of incubation at room temperature in the dark. Complete trypsinization of the protein allergens in the supernatant was done by adding 0.5 mL of trypsin (1 mg/mL in 50 mM acetic acid) and incubation at 37°C for 1 hour. Then, the digestion was stopped by adding 360 µL of 20% formic acid. The samples with the trypsinized protein allergens were usually frozen at minus 20°C before immuno-enrichment and purification.

### Example 3 - Immuno-enrichment of tryptic peptide fragments

The sample with the trypsinized protein allergens was centrifuged at 4700 × g for 20 min at 4°C. The chosen tryptic fragment from the trypsinized peptide allergen was immune-enriched, concentrated, and purified using an immunoaffinity column. For this purpose, the immunoaffinity column was initially washed twice with 3.3 mL binding buffer (20 mM sodium dihydrogen phosphate dihydrate, pH 7.0). The sample with the tryptic digest was pH-adjusted by adding 10% (v/v) 1 M Tris pH 8.5. 3.3 mL of trypsinized pH-adjusted peptide sample were added to the column at ~1 mL/min flow rate. The column was then washed twice with 3.3 mL binding buffer. The affinity-purified tryptic peptide fragment of the peptide allergen was eluted with 2 mL elution buffer (0.1 M glycine, pH 1.5). A skilled person will note that the immuno-enrichment of the tryptic peptide fragment can be arbitrarily increased by increasing the amount and/or volume of trypsinized sample added on top of the immuno-affinity column.

### Example 4 - Desalting and purification of tryptic peptides

SPE purified the tryptic peptides from the immunoaffinity step on 60-mg C18 cartridges (Phenomenex, Aschaffenburg, Germany) to prepare them for HPLC. The C18 cartridges were conditioned in 2 × 1 mL methanol (Chemsolute/Geyer, Renningen, Germany) and equilibrated in 2 × 1 mL 0.1% formic acid (FLUKA/Honeywell, Muskegon, MI, USA). The cartridge was loaded with peptides eluted from the affinity column described in example 3 and washed twice with 1 mL of 0.1% formic acid. 5 µL DMSO was added to each collector column, followed by elution with twice 0.375 mL of methanol/0.1% formic acid (95:5 v/v). The eluted tryptic peptides were dried under flowing nitrogen at 40°C and dissolved in 75 µL of 0.1% formic acid/acetonitrile (98:2 v/v). The samples were incubated at 4°C for 30 min to promote solvation. Extracts were transferred to low-protein-binding tubes (Eppendorf, Hamburg, Germany), centrifuged at 15,000 × g for 5 min at room temperature, and pipetted to HPLC glass vials for LC-MS/MS analysis.

### Example 5 -LC-MS/MS of tryptic peptides

All HPLC instrument modules were from the 1290 Infinity or Infinity II lines (Agilent Technologies, Waldbronn, Germany), including the binary pumps (G7120A) and multisampler (G7167B), flexcube^{®} (G4227A), and temperature-controlled column compartment (G7116B). 10-µL samples were injected. The peptides were separated on a bioZen 2.6 µm peptide XB-C 18 column 2.1 × 100 mm (Phenomenex) at 0.3 mL/min. Solvent A was Mili-Q water (Merck, Darmstadt, Germany) containing 0.1% formic acid, and solvent B was acetonitrile plus 0.1% formic acid. The following gradient was applied over a time of 15 min 20 s: 0-0.87 min, 98% A; 0.87-7.33 min, 98-60% A; 7.33-8.33 min, 60-2% A; 8.33-11.6 min, 2% A; 11.6-11.73 min, 2-98% A, 11.73-15.33 min, 98% A.

Peptide ions were detected using a QTRAP 6500+ triple quadrupole MS system (Sciex, Darmstadt, Germany) in positive electrospray mode with the following settings: curtain gas flow = 45 L/min, collision gas = high, source temperature = 450 °C, ion spray voltage = 5.5 kV, ion source gas 1 = 62 L/min, ion source gas 2 = 35 L/min. Tryptic peptides were used for the optimization of multiple reaction monitoring (MRM) parameters: de-clustering potential (DP), collision energy (CE), and cell exit potential (CXP). Therefore, we used the syringe pump injection mode. Automatic optimization was achieved using the Compound Optimization feature of the MS-control software Analyst v1.7.1 (Sciex). The detailed MRM method for the detection of ovalbumin. The chromatograms were interpreted using Sciex OS v17.0 (Sciex).

## Claims

1. A method for the detection and quantification of food allergens in a sample of the food and/or beverage by immunoaffinity enrichment and LC-MS/MS of one or more unique peptides from the target allergen, comprising the steps of:-
comminuting, extracting, and dissolving the sample in an aqueous extraction buffer at an elevated temperature between 40 and 95 degrees Celsius to obtain an aqueous extract of said sample, said buffer having a pH greater than 8.0 and containing greater than 2 moles per liter of a chaotropic agent selected from urea, thiourea, guanidinium chloride, guanidinium isothiocyanate, and further comprising a salt which provides inert counteranions for the dissolved peptides, amino acids, and carbohydrates, said counteranions being incapable of reacting with or precipitating calcium ions;
degradation of the polymeric carbohydrates present in the aqueous extract by adding an adequate amount of an enzyme of category E.C. 3.2.1 at an elevated temperature between 60 and 95 degrees Celsius for at least 10 minutes;
reduction of intra- und intermolecular peptidic disulfide bonds, followed by the addition of water to dilute the chaotropic agent in the extract and an adjustment of the pH of the extract in the range from 7.2 to 7.9 for enzymatic proteolysis of the peptides;
addition of an adequate amount of a protease and proteolysis of the proteinaceous components in the extract; and
immunological enrichment of one or more unique analytical peptides of the target allergen for higher sensitivity of the subsequent multiplex LC-MS/MS.

2. The method of claim 1, wherein the enzyme for the degradation of polymeric carbohydrates is of category E.C. 3.2.1.1, preferably α-amylase of *Bacillus licheniformis.*

3. The method of claim 1 or claim 2, wherein the extraction buffer contains added calcium ions for increased stability of the α-amylase.

4. The method of any one of claims 1 to 3, wherein the extraction buffer contains borate counter ions provided from a pentaborate salt, preferably from ammonium pentaborate.

5. The method of any one of claims 1 to 4, wherein the extraction buffer is a borate buffer having a pH range from 8 to 10.

6. The method of any one of claims 1 to 5, wherein the extraction buffer is free of anionic or zwitterionic detergents.

7. The method of any one of claims 1 to 6, wherein the chaotropic agent is urea.

8. The method of any one of claims 1 to 7, wherein the extraction buffer is diluted with an aqueous solution containing a buffer salt selected from ammonium bicarbonate or ammonium acetate for pH adjustment before proteolysis of the extracted protein pool.

9. The method of any one of claims 1 to 8 wherein the extracted peptide allergens are reduced and alkylated before specific proteolysis.

10. The method of any one of claims 1 to 9, wherein trypsin is used for specific proteolysis.

11. The method of any one of claims 1 to 10 wherein an alternative protease to trypsin is used for proteolysis selected from aspartic proteases, napsin-A, pepsin, presenilin, chymosin, cathepsin D/E, glutamyl endopeptidase (Glu-C), elastase, chymotrypsin, facultatively in a sequential combination with trypsin.

12. The method of any one of claims 1 to 11, wherein the immunological enrichment of the proteolytic peptides of the target allergen comprises an immunoaffinity chromatography.

13. A kit of parts for carrying out the food allergen extraction of any one of claims 1 to 12, comprising:-
a detergent-free extraction buffer having a pH of greater than 8.0, containing more than 2.0 moles per liter of a chaotropic agent selected from urea, thiourea, guanidinium chloride, guanidinium isocyanate, and a salt providing counterions for dissolved proteins, amino acids, and carbohydrates which do not react with or precipitate calcium ions; and
a solution of α-amylase of *Bacillus licheniformis.*

14. The kit of parts of claim 13, further comprising an ammonium carbonate buffer for pH adjustment of the protein extract and an active trypsin solution for sequence-specific cleavage of dissolved proteins.

15. The kit of parts of claim 13 or 14, which further comprises an immunoaffinity column for unique tryptic peptides of the selected target allergen.
